# EUROPEAN PATENT APPLICATION

(11) **EP 2 446 818 A1**
(43) Date of publication of application: **02.05.2012**
(21) Application number: 11186378.3
(22) Date of filing: 24.10.2011
(51) Int. Cl.: A61B 5/145, G01N 33/72, G01D 7/06, G06F 19/00

(54) **HbA1c measurement result display method, and display device**

(30) Priority: 27.10.2010 JP 2010240856
(71) Applicant: Arkray, Inc., Minami-ku Kyoto-shi Kyoto 601-8045 (JP)
(72) Inventor: Sugiyama, Koji, Kyoto, Kyoto 602-0008 (JP)
(74) Representative: MacDougall, Alan John Shaw

(57) **Abstract**

A method for displaying HbA1c measurement results is provided, which makes it possible to prevent overlooking a sample that exhibits an HbAlc value at a predetermined level different from a normal value, for example, a sample that exhibits a diabetic type. The method is a display method for displaying spectrum data as results of measurement of hemoglobin A1c (HbAlc) in a sample by separation analysis, and the method includes applying one of at least two different types of designs to a peak area for HbAlc in the spectrum data, according to an amount of HbAlc in the sample, or according to the amount of HbAlc and an amount of blood glucose in the sample.

## Description

### Technical Field

The present invention relates to a display method, and a display device for displaying HbAlc measurement results.

### Background Art

A device for measuring hemoglobin A1c (HbA1c) based on separation analysis, such as, for example, HPLC (high performance liquid chromatography), as the measurement principle, often outputs a separation chromatogram together with an HbAlc value as measurement results. In the chromatogram, however, there are peaks other than the peak of HbAlc as a target object. Therefore, the HbAlc fraction (peak area) on the chromatogram is hatched sometimes so as to be more distinguishable from the other Hb fractions (Takuji MURAKAMI et al., "Development of Tosoh's fully automated glycohemoglobin analyzer HLC-723 G8 ", TOSOH Research & Technology Review Vol. 50 (2006), pp. 69-72).

On the other hand, in Japan, new diagnostic criteria for diabetes were put into effect on July 1, 2010, and the HbAlc value was added to diagnosis items. In other words, hereafter in clinical practices, the HbAlc value plays a role not only as a treatment marker for diabetes as conventionally but also as a diagnosis marker for diabetes.

### Summary of the Invention

### Problem to be Solved by the Invention

Regarding the HbAlc value as a treatment marker for diabetes, comparison between a previously measured value and a currently measured value of the same has important implications for checking treatment effects and deciding a new treatment policy. On the other hand, regarding the HbAlc value as a diagnosis marker for diabetes, the focus is which category the value falls in, i.e., the diabetic type or the non-diabetic type, according to the Diagnostic Criteria for Diabetes. In clinical practices where many samples are measured, elimination of overlooking of diabetes-type samples is required.

The present invention provides a display method, a display device, and a display program for displaying HbAlc measurement results, which make it possible to prevent the overlooking of a sample exhibiting a predetermined HbAlc value different from a normal HbAlc value, for example, a sample exhibiting the diabetic type.

The present invention in one aspect relates to a method for displaying spectrum data as results of measurement of hemoglobin A1c (HbA1c) in a sample by separation analysis, the method including applying one of at least two different types of designs to a peak area for HbAlc in the spectrum data, according to the amount of HbAlc in the sample, or according to the amount of HbAlc and the amount of blood glucose in the sample.

The present invention in another aspect relates to a device including: a measurement section that measures hemoglobin A1c (HbA1c) in a sample by separation analysis and obtains a measured amount of HbAlc and spectrum data; a display data conversion section that applies one of at least two different types of designs to a peak area for HbAlc in the spectrum data according to the amount of HbAlc or according to the amount of HbAlc and the amount of blood glucose; and at least one of a display section that displays data obtained through the conversion, a printing section that prints out the data, and a transmission section that transmits the data.

The present invention in still another aspect relates to a display program that causes a processor that displays an amount of hemoglobin A1c (HbAlc) and spectrum data obtained by measuring HbAlc in a sample by separation analysis to execute: a process of making access to a recording section that has preliminarily recorded correspondence relationship data indicating correspondence relationship between either the amount of HbAlc or the amount of HbAlc and the amount of blood glucose on one hand and a design on the other hand, and deciding a design corresponding to either the amount of HbAlc in the sample or the amount of HbAlc and the amount of blood glucose; a process of altering a design of a peak area for HbAlc in the spectrum data of the sample to the decided design; and a process of displaying, printing, or outputting to outside, the spectrum data having the alteration.

### Effects of the Invention

With the present invention, it is possible to prevent overlooking measurement results that should be noticed, such as in HbAlc measurement using separation analysis.

### Brief Description of the Drawings

[FIG. 1] FIG. 1 is a functional block diagram showing an exemplary configuration of a device according to an embodiment.
[FIG. 2] FIG. 2 is a functional block diagram showing another exemplary configuration of a device according to the embodiment.
[FIG. 3] FIG. 3 is a flowchart showing an exemplary action of a display device.
[FIG. 4] FIG. 4 is a flowchart showing another exemplary action of the display device.
[FIG. 5] FIG. 5A shows an exemplary chromatogram displayed, and FIG. 5B shows another exemplary chromatogram displayed.

### Detailed Description of the Invention

In the present specification, "hemoglobin A1c (HbAlc)" refers to hemoglobin having glucose bonded to an N terminal of a β chain thereof, which is one of protein components in erythrocytes, and unless otherwise provided, it refers to stable HbAlc (s-HbA1c). Examples of components other than the stable HbAlc that can be detected when blood or a blood-derived sample is analyzed by separation analysis include, but not limited to, hemoglobin A0 (HbA0), hemoglobin A1a (HbA1a), hemoglobin Alb (HbAlb), hemoglobin A2 (HbA2), hemoglobin S (HbS, sickle cell hemoglobin), hemoglobin F (HbF, fetal hemoglobin), hemoglobin M (HbM), hemoglobin C (HbC), hemoglobin D (HbD), hemoglobin E (HbE), met-hemoglobin, carbamoylated hemoglobin, acetylated hemoglobin, and unstable HbAlc (1-HbA1c).

In the present specification, the "sample" refers to a sample prepared from a sample material, or the sample material per se. Examples of the sample materials include biological samples containing components derived from living bodies. The sample material is preferably a material containing the above-described target object, such as a sample containing hemoglobin. Examples of the biological sample include, but not limited to, blood, blood-derived materials containing erythrocytes, saliva, and cerebrospinal fluid. Blood is, for example, blood sampled from a living body. The blood-derived material containing erythrocytes is, for example, a material that is separated or prepared from blood and contains erythrocytes, and examples of the same include a fraction of blood cells obtained by removing plasma, concentrate of blood cells, freeze-dried blood or blood cells, a hemolyzed sample obtained by hemolyzing whole blood, centrifuged blood, blood samples obtained through spontaneous sedimentation, and washed blood cells.

In the present specification, the "amount of HbAlc" refers to a concentration of HbAlc in a sample or blood, and from a viewpoint of practical application, it preferably refers to an HbAlc value. In the present specification, the "HbAlc value" refers to a ratio of HbAlc with respect to hemoglobin in a sample or blood (concentration of HbAlc / concentration of hemoglobin), and the unit thereof is %.

On the other hand, the HbAlc value (JDS value) used in Japan is characterized in that it has been standardized in Japan earlier than in other countries, but it has a problem in that it is about 0.4 % lower than the HbAlc value used in many countries in the world (NGSP value). The Japan Diabetes Society therefore decided that a new HbAlc value obtained by adding 0.4 % to the conventional HbAlc value expressed in the JDS value style is referred to as an international standard value, and the diabetes diagnosis is performed, with reference to the JDS value at the moment, and with reference to the international standard value after a date of implementation of the international standard value that will be made public later.

According to the new diagnostic criteria for diabetes revised on July 1, 2010, diabetes is diagnosed into the following three categories based on the HbAlc value and the blood glucose value:
a) fasting blood glucose value > 126 mg/dl, and HbAlc value > 6.5 % (international standard value) (JSD value: 6.1 %);
b) blood glucose value 2 hours after OGTT ≥ 200 mg/dl, and HbAlc value > 6.5 % (international standard value) (JSD value: 6.1 %); and
c) casual blood glucose value > 200 mg/dl, and HbAlc value > 6.5 % (international standard value) (JSD value: 6.1 %).
Here, "OGTT" represents "oral glucose tolerance test".

Diabetes is a group of disorders characterized by a chronic hyperglycemia state due to insufficient effect of insulin, which finally lead to death if sustainable hyperglycemia state continues for a long time and causes diabetic complications. Diabetes treatments focus on preventing complications from developing, and therapeutic intervention at an early stage of diabetes and continuous maintenance and control with suppression of increase of a blood glucose value has a great significance. Therefore, in order to start treatments for diabetes at an early stage, health checkup, medical tests on patients with suspected diabetes, etc. are particularly important.

The present invention focuses attention on an increase in the possibility that a sample to be classified as diabetes or a sample to be classified as suspected diabetes could be overlooked in clinical sites where the HbAlc value is used as a diagnosis marker for diabetes. The present invention is based on knowledge that such overlooking can be reduced easily by applying a different design to a peak area for HbAlc in a chromatogram displayed or printed for each measurement, depending on which type a sample measured belongs to, for example, the "diabetic type" and the "non-diabetic type".

More specifically, the present invention in one aspect relates to a method for displaying spectrum data as a result of measurement of hemoglobin A1c (HbA1c) in a sample by separation analysis (hereinafter this method is referred to as "the display method of the present invention" also), the method including applying one of at least two different types of designs to a peak area for HbAlc in the spectrum data, according to the amount of HbAlc in the sample, or according to the amount of HbAlc and the amount of blood glucose in the sample.

With the display method of the present invention, it is possible to achieve an effect of, for example, reducing the overlooking of a sample that indicates an HbAlc value that should be noticed, in a clinical site where many samples are measured. It should be noted that the display method of the present invention does not include making a diagnosis with respect to an individual, for example, making a diagnosis for diabetes with respect to an individual who has provided a sample. The display method of the present invention relates to displaying a classification of a sample, and is able to provide a guideline or information for diagnosis carried out by a doctor. The display method of the present invention, however, is not limited to the purpose of diagnosis.

In the present specification, the "separation analysis" refers to a method that is for performing an analysis while individually separating a target object contained in a sample, and that is capable of performing separation analysis of HbAlc. Specific examples of the separation analysis include liquid chromatography, electrophoresis, gas chromatography, and electrochromatography. Liquid chromatography is, particularly, high performance liquid chromatography (HPLC), and, for example, cation exchange chromatography, anion exchange chromatography, partition chromatography, reversed-phase partition chromatography, or gel filtration chromatography. Examples of the electrophoresis include, particularly, capillary electrophoresis (CE), for example, capillary zone electrophoresis, capillary isoelectric focusing electrophoresis, capillary electrokinetic chromatography, and capillary gel electrophoresis.

In the present specification, "spectrum data" refer to a waveform graph representing a separation state of components including HbAlc in a sample. "Spectrum data" refer to a chromatogram when the separation analysis is chromatography, while the same refer to an electropherogram when the separation analysis is capillary electrophoresis. In the present specification, a "peak area for HbAlc" refers to an area occupied by HbAlc in spectrum data, i.e., an HbAlc fraction.

In the present specification, a "design" refers to a marking, a pattern, a color, and a combination thereof applied to a peak area for HbAlc when spectrum data are displayed or printed. Examples of the designs that can be applied to a peak area include a colorless and patternless one. Those skilled in the art are able to suitably select a combination of two or more types of designs that can be easily distinguished.

In an embodiment of the present invention, the type of the design applied to a peak area for HbAlc is altered with reference to the HbAlc value as the diagnostic criteria for diabetes. The HbAlc value as the diagnostic criteria for diabetes is, for example, the international standard value of not less than 6.5 % (JDS value: not less than 6.1 %), as described above. It should be noted that when the diagnostic criteria for diabetes are revised as to the aforementioned value, the value after the revision may be used as the reference value.

In still another embodiment of the present invention, the display may be switched with reference to the blood glucose value in addition to the HbAlc value, following the new diagnostic criteria for diabetes in Japan. In the present embodiment, two designs may be used as designs for the "diabetic type" and the "non-diabetic type" applied to one peak area for HbAlc. More specifically, if data fall under any one of the following conditions a) to c) as described above, the design for the "diabetic type" is applied to the peak area; and if data do not fall under any of the following conditions, the design for the "non-diabetic type" is applied to the peak area. Further, different designs may be applied as the "diabetic type" design, depending on which one of the following conditions a) to c) the data fall under.
a) fasting blood glucose value > 126 mg/dl, and HbAlc value > 6.5 % (international standard value) (JSD value: 6.1 %);
b) blood glucose value 2 hours after OGTT ≥ 200 mg/dl, and HbAlc value > 6.5 % (international standard value) (JSD value: 6.1 %); and
c) casual blood glucose value > 200 mg/dl, and HbAlc value > 6.5 % (international standard value) (JSD value: 6.1 %).

Information on the blood glucose value is preferably based on one sample. The blood glucose value may be measured by a glucose measurement unit incorporated in a device for measuring the amount of HbAlc, or may be measured by a measurement device other than the device for measuring the amount of HbAlc.

The form of display in the display method of the present invention may be the same as the display by a conventional HbAlc measurement device, and examples of the same include printing by a printer, display by a display device or the like, and electrical output for communication or to an electronic medium. The form of display may be selected appropriately from these.

Next, a device of the present invention is explained.

The present invention in one aspect relates to a device that includes a measurement section that measures HbAlc in a sample by separation analysis and obtains a measured amount of HbAlc and spectrum data; a display data conversion section that applies one of at least two different types of designs to a peak area for HbAlc in the spectrum data according to the amount of HbAlc or according to the amount of HbAlc and an amount of blood glucose; and at least one of a display section that displays display data obtained through the conversion, a printing section that prints out the data, and a transmission section that transmits the data to outside. With the device of the present aspect, the measurement of HbAlc and the display by the display method of the present invention can be performed.

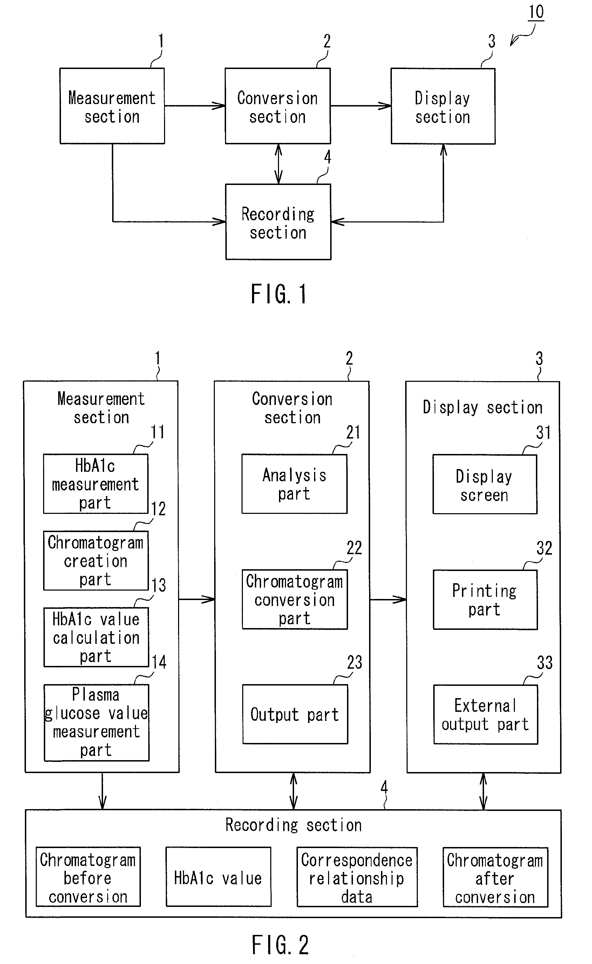

The device of the present aspect is explained with reference to an embodiment shown in FIG. 1. FIG. 1 is a functional block diagram showing an exemplary configuration of an embodiment of the device of the present aspect. A device 10 shown in FIG. 1 is an HbAlc measurement device that uses separation analysis, and also is a device that performs the display method of the present invention. The device 10 includes a measurement section 1, a conversion section 2, a display section 3, and a recording section 4. The measurement section 1 performs HbAlc measurement using separation analysis. The creation and correction of spectrum data and the calculation of an HbAlc value from the obtained measurement data may be performed by the measurement section 1, or by the conversion section 2. The conversion section 2 converts spectrum data so that one of at least two different types of designs is applied to a peak area for HbAlc in the spectrum data according to the spectrum data and the HbAlc value thus obtained. The spectrum data converted are displayed by the display section 3. As to a specific conversion method carried out by the conversion section 2, the display method of the present invention described above may be referred to.

In the case where the blood glucose value is used by the conversion section 2, the measurement section 1 may include a blood glucose value measurement part (e.g., a glucose measurement unit). Alternatively, data measured by an external measurement device may be used. In such a case, the device 10 includes an I/O port such as a communication port, a keyboard, or a barcode reader, and a blood glucose value can be entered through the I/O port. The display section 3 may be a visual display means such as a liquid crystal display device, an organic electroluminescence display device, or a printer, or may be an electric output such as a communication port, an I/O port, or an electronic medium. The recording section 4 is not an essential component for the device of the present aspect, but it can record, for example, correspondence relationship data that indicate correspondence relationship between the amount of HbAlc and designs, and/or correspondence relationship between the amount of HbAlc and the blood glucose value on one hand and designs on the other hand.

FIG. 2 shows another embodiment of the device 10. FIG. 2 illustrates exemplary configurations of the measurement section 1, the conversion section 2, and the display section 3, as well as exemplary data recorded in the recording section 4. The measurement section 1 includes an HbAlc measurement part 11, a chromatogram creation part 12, an HbAlc value calculation part 13, and a blood glucose value measurement part 14. The conversion section 2 includes an analysis part 21, a chromatogram conversion part 22, and an output part 23. The display section 3 includes a display screen 31, a printing part 32, and an external output part 33. The recording section 4 records, for example, a chromatogram before conversion, an HbAlc value, correspondence relationship data, and a chromatogram after conversion.

A sample is measured by the HbAlc measurement part 11 of the measurement section 1, and measurement data are obtained. A chromatogram is created by the chromatogram creation part 12 using the measurement data, and an HbAlc value is calculated from the chromatogram by the HbAlc value calculation part 13. The chromatogram and the HbAlc value are recorded in the recording section 4. Further, a blood glucose value of the same sample is measured by the blood glucose value measurement part 14 and recorded in the recording section 4 as required. Next, the analysis part 21 of the conversion section 2 decides a design to be applied to a peak area for HbAlc , based on the HbAlc value obtained by the measurement section 1, and correspondence relationship data obtained by access to the recording section 4. The chromatogram conversion part 22 converts the chromatogram by applying the decided design to the peak area for HbAlc in the chromatogram obtained by the measurement section 1. The converted chromatogram is recorded in the recording section 4. The converted chromatogram is fed via the output part 23 to the display section 3. The display section 3 outputs the converted chromatogram to the display screen 31, the printing part 32, and/or the external output part 33. The display section 3 is capable of displaying, printing, or outputting to outside the recorded chromatogram after conversion, by making access to the recording section 4 as required.

The function of the conversion section 2 shown in FIGS.1 and 2 can be realized by, for example, a processor executing a predetermined program, the processor being incorporated in a device of the present aspect. The recording section 4 can be composed of a recording device such as a memory or a HDD accessible from the processor.

Therefore, the present invention in an aspect is a display program that causes a processor that displays an amount of hemoglobin A1c (HbAlc) and spectrum data obtained by measuring HbAlc in a sample by separation analysis to executes a process of making access to a recording section that has preliminarily recorded correspondence relationship data indicating correspondence relationship between either the amount of HbAlc or the amount of HbAlc and the amount of blood glucose on one hand and a design on the other hand, and deciding a design corresponding to either the amount of HbAlc in the sample or the amount of HbAlc and the amount of blood glucose; a process of altering a design of a peak area for HbAlc in the spectrum data of the sample to the decided design; and a process of displaying, printing, or outputting to outside, the spectrum data having the alteration.

The device 10 shown in FIGS. 1 and 2 may have such a configuration that the measurement section 1, the conversion section 2, the display section 3, and the recording section 4 are integrally provided, and alternatively, such a configuration that a display device including the conversion section 2, the display section 3, and the recording section 4 is connected with the measurement section 1 that is independently provided.

Therefore, the present invention in an aspect relates to a device that includes a reception section that obtains an amount of HbAlc and spectrum data as results of measurement of HbAlc in a sample by separation analysis; a display data conversion section that applies one of at least two different types of designs to a peak area for HbAlc in the spectrum data according to the amount of HbAlc or according to the amount of HbAlc and an amount of blood glucose; and at least one of a display section that displays display data obtained through the conversion, a printing section that prints out the data, and a transmission section that transmits the data to outside.

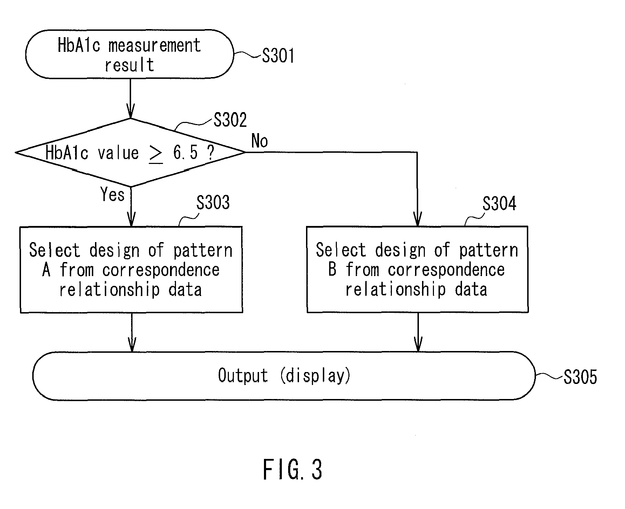

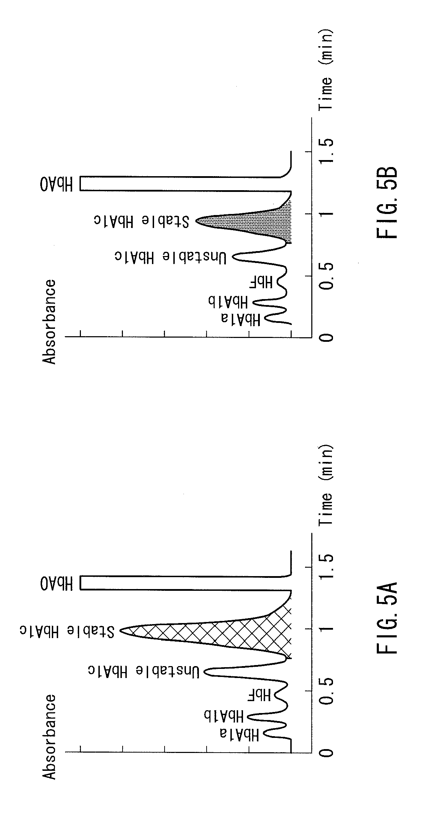

FIG. 3 is a flowchart showing an exemplary action of the device 10 according to the embodiment shown in FIG. 1. The measurement section 1 performs HbAlc measurement (S301). Next, the conversion section 2 decides a design to be applied to a peak area for HbAlc based on an HbAlc value obtained by the measurement (S302). If the HbAlc value is 6.5 % or more (international standard value, (JSD value: 6.1 % or more)), a pattern A recorded in the recording section 4 is selected and the chromatogram is converted (S303). Further, if the HbAlc value is less than 6.5 % (international standard value, (JSD value: less than 6.1 %)), a pattern B recorded in the recording section 4 is selected and the chromatogram is converted (S304). The chromatogram after conversion is displayed by the display section 3 (S305). An exemplary chromatogram in which the patternAis applied is shown in FIG. 5A, and an exemplary chromatogram in which the pattern B is applied is shown in FIG. 5B.

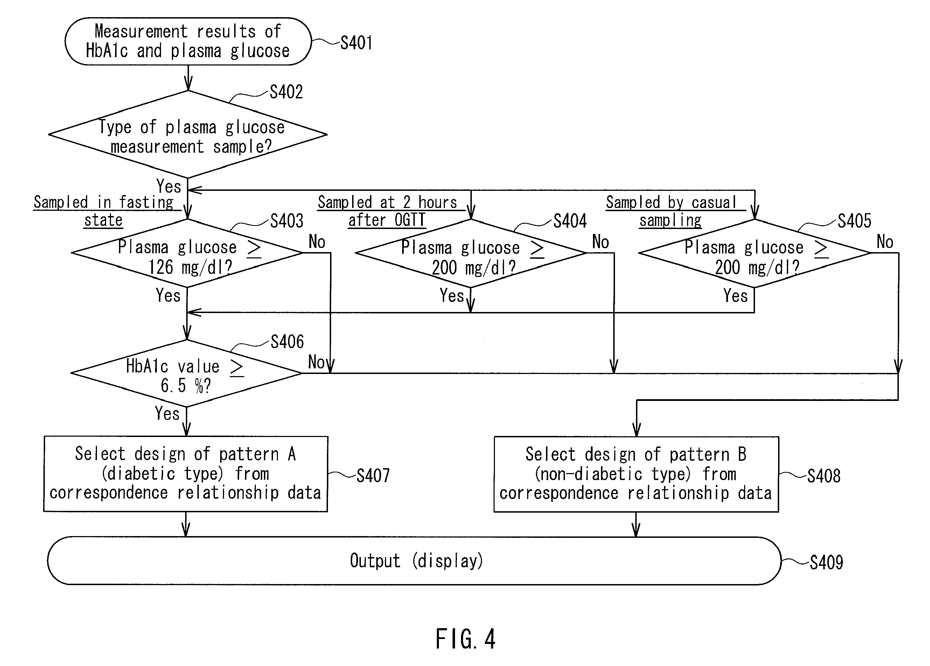

FIG. 4 is a flowchart showing an exemplary action of the device 10 according to an embodiment shown in FIG. 2. Measurement is carried out with respect to a sample in the HbAlc measurement part 11 and the blood glucose value measurement part 14 (S401). Next, a state of the sample when it was sampled is determined by the analysis part 21 (S402). If it was sampled in the fasting state, the flow goes to S403; if it was sampled at 2 hours after OGTT, the flow goes to S404; and if it was sampled by casual sampling, the flow goes to S405. If it is determined that the sample exhibits a blood glucose value of not less than a predetermined level at each of the three steps, the flow goes to S406. At S406, a processing based on an HbAlc value is carried out. More specifically, if the sample exhibits an HbAlc value of not less than 6.5 %, the pattern A (diabetic type) recorded in the recording section 4 is selected and the chromatogram is converted (S407). If the sample exhibits an HbAlc value of less than 6.5 % (international standard value, (JSD value: less than 6.1 %)), the pattern B (non-diabetic type) recorded in the recording section 4 is selected, and the chromatogram is converted (S408). Further, if the sample exhibits a blood glucose value of less than a predetermined level at S403, S404, and S405 and exhibits an HbAlc value of less than 6.5 % (international standard value, (JSD value: less than 6.1 %)), the pattern B (non-diabetic type) recorded in the recording section 4 is selected, and the chromatogram is converted (S408). The chromatogram after conversion is displayed by the display section 3 (S409). An exemplary chromatogram in which the pattern Ais applied is shown in FIG. 5A, and an exemplary chromatogram in which the pattern B is applied is shown in FIG. 5B.

### Industrial Applicability

The present invention is useful in, for example, the fields of medical diagnostic equipment, clinical inspection equipment, etc.

## Claims

1. A method for displaying spectrum data as a result of measurement of hemoglobin A1c (HbAlc) in a sample by separation analysis, the method comprising:
applying one of at least two different types of designs to a peak area for HbAlc in the spectrum data, according to an amount of HbAlc in the sample, or according to the amount of HbAlc and an amount of blood glucose in the sample.

2. The method according to claim 1, wherein the type of the design applied to the peak area for HbAlc is altered with reference to an HbAlc value as diagnostic criteria for diabetes.

3. The method according to claim 1 or 2, wherein the type of the design applied to the peak area for HbAlc when the sample exhibits an HbAlc value as the international standard value of 6.5 % or more (JDS value: 6.1 % or more), and the type of the design applied to the peak area for HbAlc when the sample exhibits an HbAlc value as the international standard value of less than 6.5 % (JDS value: less than 6.1 %), are different from each other.

4. The method according to any one of claims 1 to 3, wherein either a design for a diabetic type or a design for a non-diabetic type is applied to the peak area for HbAlc, with reference to an HbAlc value and a blood glucose value as diagnostic criteria for diabetes.

5. The method according to any one of claims 1 to 4, wherein a design for a diabetic type is applied to the peak area for HbAlc, when any one of conditions a) to c) shown below is satisfied:
a) fasting blood glucose value > 126 mg/dl, and HbAlc value > 6.5 % (international standard value) (JSD value: 6.1 %);
b) blood glucose value 2 hours after OGTT ≥ 200 mg/dl, and HbAlc value > 6.5 % (international standard value) (JSD value: 6.1 %); and
c) casual blood glucose value > 200 mg/dl, and HbAlc value > 6.5 % (international standard value) (JSD value: 6.1 %).

6. A device comprising:
a measurement section that measures hemoglobin A1c (HbAlc) in a sample by separation analysis and obtains a measured amount of HbAlc and spectrum data;
a display data conversion section that applies one of at least two different types of designs to a peak area for HbAlc in the spectrum data according to the amount of HbAlc or according to the amount of HbAlc and an amount of blood glucose; and
at least one of a display section that displays display data obtained through the conversion, a printing section that prints out the data, and a transmission section that transmits the data.

7. A device comprising:
a reception section that obtains an amount of HbAlc and spectrum data as results of measurement of hemoglobin A1c (HbAlc) in a sample by separation analysis;
a display data conversion section that applies one of at least two different types of designs to a peak area for HbAlc in the spectrum data according to the amount of HbAlc or according to the amount of HbAlc and an amount of blood glucose; and
at least one of a display section that displays display data obtained through the conversion, a printing section that prints out the data, and a transmission section that transmits the data to outside.

8. The device according to claim 6 or 7, wherein the display data conversion section alters the type of the design applied to the peak area for HbAlc, by referring to the HbAlc as the diagnostic criteria for diabetes.

9. The device according to any one of claims 6 to 8, wherein the display data conversion section applies different types of the designs to the peak area for HbAlc, respectively, when the sample exhibits an HbAlc value as the international standard value of 6.5 % or more (JDS value: 6.1 % or more), and when the sample exhibits an HbAlc value as the international standard value of less than 6.5 % (JDS value: less than 6.1 %).

10. The device according to any one of claims 6 to 9, the display data conversion section applies either a design for a diabetic type or a design for a non-diabetic type to the peak area for HbAlc, by referring to an HbAlc value and a blood glucose value as diagnostic criteria for diabetes.

11. The device according to any one of claims 6 to 10 wherein the display data conversion section applies a design for a diabetic type to the peak area for HbAlc, when any one of conditions a) to c) shown below is satisfied:
a) fasting blood glucose value > 126 mg/dl, and HbAlc value > 6.5 % (international standard value) (JSD value: 6.1 %);
b) blood glucose value 2 hours after OGTT ≥ 200 mg/dl, and HbAlc value > 6.5 % (international standard value) (JSD value: 6.1 %); and
c) casual blood glucose value > 200 mg/dl, and HbAlc value > 6.5 % (international standard value) (JSD value: 6.1 %).

12. The device according to any one of claims 6 to 11, wherein the separation analysis is liquid chromatography, electrophoresis, gas chromatography, or electrochromatography, and the spectrum data are data of a chromatogram or an electropherogram.

13. A display program that causes a processor that displays an amount of hemoglobin A1c (HbA1c) and spectrum data obtained by measuring HbAlc in a sample by separation analysis to execute the processes of
making access to a recording section that has preliminarily recorded correspondence relationship data indicating correspondence relationship between either the amount of HbAlc or the amount of HbAlc and an amount of blood glucose on one hand and a design on the other hand, and deciding a design corresponding to either the amount of HbAlc in the sample or the amount of HbAlc and the amount of blood glucose in the sample;
altering a design of a peak area for HbAlc in the spectrum data of the sample to the decided design; and
displaying, printing, or outputting to outside, the spectrum data having the alteration.
